# EUROPEAN PATENT SPECIFICATION

(11) **EP 1 292 258 B1**
(45) Date of publication and mention of the grant of the patent: **12.01.2005**
(21) Application number: 01938900.6
(22) Date of filing: 05.06.2001
(51) Int. Cl.: A61F 13/42

(54) **ABSORBENT ARTICLE**
ABSORBIERENDER ARTIKEL
ARTICLE ABSORBANT

(30) Priority: 13.06.2000 SE 0002206
(43) Date of publication of application: 19.03.2003
(73) Proprietor: SCA Hygiene Products AB, 405 03 Göteborg (SE)
(72) Inventor: STENBERG, Anders, S-439 92 Onsala (SE)
(74) Representative: Egeröd, Lisbeth
(86) International application number: PCT/SE2001/001250
(87) International publication number: WO 2001/095845

(56) References cited:
- EP-A1- 0 951 889
- EP-A2- 0 496 567
- EP-A2- 0 925 769
- WO-A1-99/16401
- FR-A1- 2 711 317
- US-A- 4 231 370
- US-A- 4 662 875

## Description

### Technical field

The present invention refers to an absorbent article such as a diaper or incontinence guard comprising a liquid pervious topsheet, a liquid impervious backsheet and an absorbent body enclosed therebetween, at which on the inside of the liquid impervious backsheet, i e on the side adjacent the absorbent body, there is arranged a wetness indicator in a certain pattern, which is visible through the backsheet material.

### Background of the invention

Wetness indicators especially on diapers and incontinence guards for adult incontinent persons, have been known for several years and facilitates for the nursing staff to determine whether the diaper or incontinence guard needs to be changed. It is for example known through EP-A-0 211 524 to print a pattern of a water soluble dye on the inside of the backsheet material of a diaper by means of ink beam technique. The dyestuff can alternatively be of a type that changes colour or tint when brought into contact with urine. The printed pattern can also consist of symbols or codes for quality- or other manufacturing control.

It is further known through EP-A-0 203 715 to treat an absorbent body of a diaper so that it gets a tight paperlike structure on the backside, which then is used as a reception surface for a wetness indicating dye applied in a pattern that is visible through the backside material of the diaper.

It is also known to have capillary wetness indicators, i e a thin strip or thread having a high capillary effect arranged in connection to the absorption body and which is in contact with an indicator which dissolves, changes colour or tint when brought in contact with a liquid. Such wetness indicators are known through for example US-A 4,738,674 and GB-A-2 327 354.

The wetness indicator can be arranged at different locations, such as along the side of the absorption body or on the underside thereof. It can be therefore be difficult for the nurse to locate the wetness indicator on the article in order to read it to see whether the article needs to be changed or not.

WO 99/16401 discloses an diaper having a wetness indicator on the inside of the backsheet material. The backsheet material is a laminate of a fibrous nonwoven material and a polymer sheet. A plurality of translucent windows are arranged in said backsheet through which the wetness indicator is visible.

US-A- 4,231,370 discloses a diaper having a wetness indicator in the form of a coating applied in a pattern on the inside of a translucent backsheet material.

FR-A- 2 711 317 discloses a wetness indicator comprising an inner portion which changes colour when it is wetted by urine and an outer portion which is translucent plastic material.

EP-A-0 925 769 discloses a diaper having a backsheet with transparent zones through which a wetness of the absorbent core can be observed.

### Object and most important features of the invention

The object of the present invention is to make it easier for the nurse to identify the location of the wetness indicator on the article and by that facilitate the reading thereof. This has been solved by applying the wetness indicator on or adjacent at least one strip having a colour or tint different from the rest of the backsheet material.

According to one embodiment the strip is separate from the backsheet material and is applied to the inside thereof.

According to another embodiment the strip is a part of the backsheet material which is coloured in another colour or tint than the rest of the backsheet material.

According to a preferred embodiment of the invention the colour or tint of the strip is an indication of the type, size, absorption capacity or the like of the article.

According to an embodiment the strip extends in the longitudinal direction of the article over the entire or at least over a substantial part thereof.

According to another embodiment the strip extends in the transverse direction of the article and is applied opposite the part of the article that is intended to form a fold in the folded packaging position of the article.

The width of the strip should be between 1 and 8 cm, preferably between 2 and 7 cm and more preferably between 3 and 6 cm.

On the strip or adjacent thereto there can further be printed symbols, codes or the like indication the type of product, size, absorption capacity or the like.

### Description of the drawings

The invention will in the following be closer described with reference to some embodiments shown in the accompanying drawings.
Fig. 1 is a plan view of a first embodiment of a diaper seen from the underside.
Fig. 2 is a section according to the line II-II in Fig. 1.
Fig. 3 is a plan view of another embodiment of an incontinence guard seen from the back side.
Fig. 4 shows in perspective a diaper in folded condition.
Fig. 5 is a plan view of a further embodiment of an incontinence guard seen from the backside.

### Description of embodiments

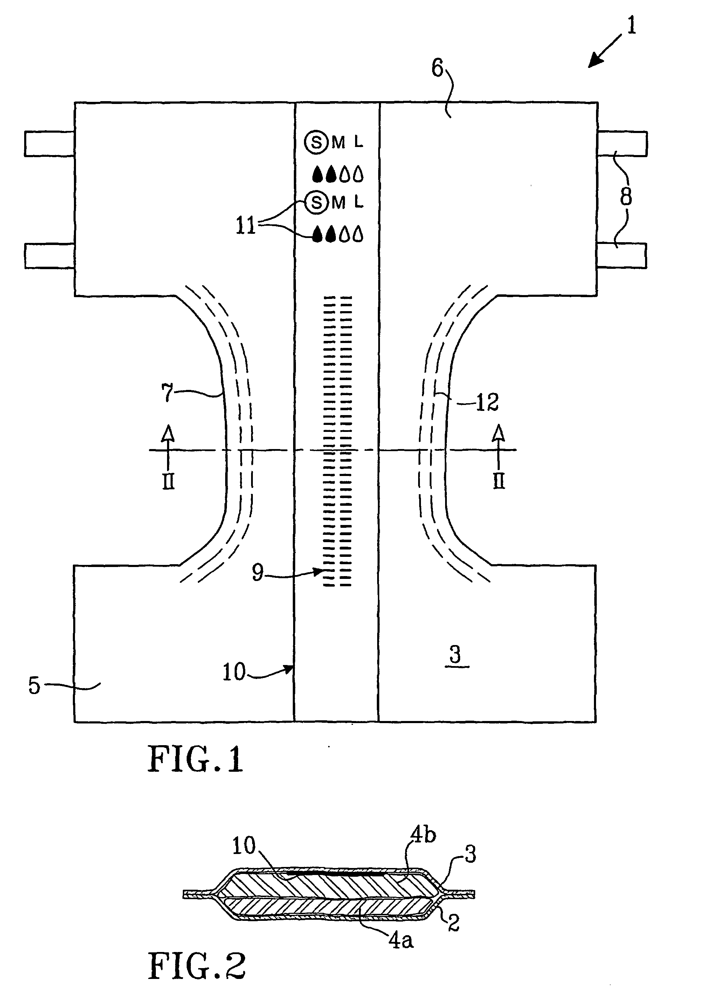

In Fig. 1 and 2 there is disclosed an embodiment of a diaper 1, comprising a liquid pervious topsheet 2, a liquid impervious backsheet 3 and an absorbent body 4 enclosed therebetween. The absorbent body 4 can comprise two or more layers, such as liquid acquisition layer 4a and storage layer 4b.

The liquid pervious topsheet 2 can be a nonwoven material, for example a spunbond material of synthetic filaments, a meltblown material, a thermobonded material or a bonded carded fibrous web.

The liquid impervious backsheet material 3 can consist of a plastic film, a nonwoven material coated with a liquid impervious material or a hydrophobic nonwoven material which resists liquid penetration. The backsheet material can be a so called breathable material, which allows penetration of water vapour but prevents passage of liquid penetration. It may in this case be a porous plastic film, a nonwoven material or a laminate of a porous plastic film and a nonwoven material.

The topsheet 2 and the backsheet 3 have a somewhat larger extension in the plane than the absorbent body 4 and extend outside the edges thereof. The layers 2 and 3 are connected within their projecting portions, for example by gluing or welding with ultra sonic or heat.

The absorbent body 4 can be of any conventional kind. Examples of commonly occurring absorption materials are cellulosic fluff pulp, tissue layers, highly absorbent polymers (so called superabsorbents), absorbent foam materials, absorbent nonwoven materials and the like. It is common to combine cellulosic fluff pulp with superabsorbents in an absorbent body. It is also common to have absorbent bodies comprising layers of different materials with different properties with respect to liquid acquisition capacity, distribution capacity and storage capacity. This is well-known to the person skilled in the art and need therefor not be described in detail. The thin absorbent bodies that are common in for example baby diapers and incontinence guards often comprise a liquid storage layer 4b in the form of a compressed mixed or layered structure of cellulosic fluff pulp and superabsorbent and a liquid acquisition layer 4a arranged on top of the liquid storage layer, said liquid acquisition layer having an open porous structure with the ability to quickly acquire the discharged body fluid and temporarily store it before it is absorbed by the underlying storage layer 4b.

The diaper/incontinence guard is intended to enclose the lower part of the wearer's torso as a pair of absorbent pants. It is provided with a front portion 5 intended during use to be turned forwards on the user, and a rear portion 6 intended during use to be turned backwards on the user, and a narrower crotch portion 7 located between the front and rear portion, said crotch portion is intended to be applied in the crotch of the wearer between the legs. The rear portion 6 is provided with a pair of tape flaps 8 or other type of fastening means such as hook and loop type, and which are intended to be used for fasten together the diaper to the desired pantlike shape. Alternatively the fastening means can be arranged at the front portion. Around the leg portions there are arranged elastic threads 12 or the like, which provide a sealing effect around the thighs of the wearer.

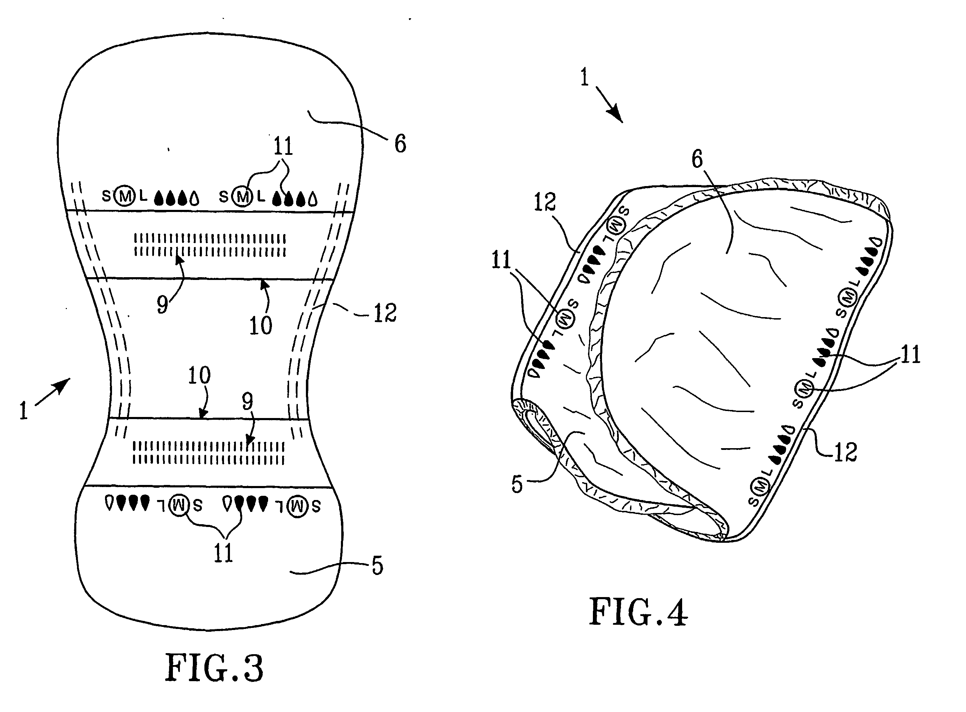

In Fig. 3-5 there are shown examples of an incontinence guard intended to be kept in place by a pair of tight pants. The fastening means in the form of tapes or hooks and loops are therefore lacking. Possibly there can on the underside of the backsheet material be applied glue strings or another kind of friction increasing means in order to keep the incontinence guard in place in the pants.

It is pointed out that the incontinence guard and the diaper shown in the drawings and described above only represent a couple of examples of absorbent articles and are by no means limiting for the present invention. Thus the shape of the article as well as its overall design can be varied. The absorbent article can also be a pant diaper, a belt-provided diaper or the like.

On the inside of the backsheet material 3 there is applied a wetness indicator 9, which preferably consists of a dyestuff which dissolves or changes colour or tint when brought into contact with liquid, and which is printed directly on the backsheet material or alternatively on a separate strip that is fastened to the inside of the backsheet material.

The wetness indicator 9 is applied on or adjacent at least one strip 10 with a colour or tint that is different the rest of the backsheet material. The strip 10 either consists of a part that is separate from the backsheet material 3 and is fastened to the inside thereof, or consists of a part of the backsheet material that is coloured in another colour or tint than the rest of the backsheet material. The strip 10 can consist of one single coloured strip or of a striped pattern or other optional pattern. The strip 10 may either be a continuous strip or may consist of several discontinuous portions arranged so as to form a discontinuous line. The strip 10 may also comprise two or more strips arranged close to each other.

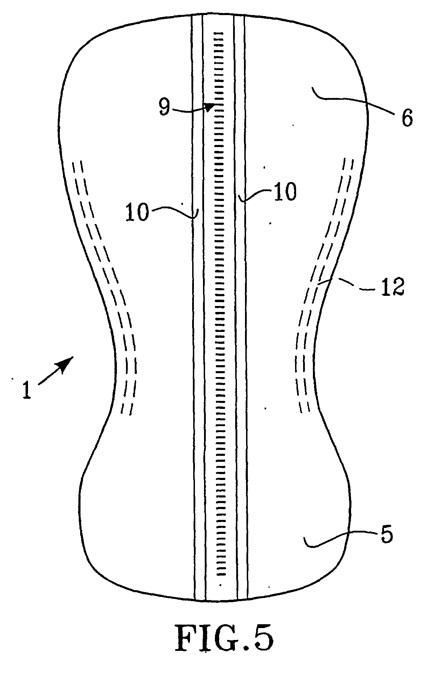

In the embodiments shown in Figs. 1-4 the wetness indicator 9 is applied on the strip 10, while in the embodiment shown in Fig. 5 it is applied between two strips 10 extending substantially in parallel.

According to a further embodiment the wetness indicator 9 may be visible through apertures or windows arranged in the strip 10.

By the fact that the wetness indicator 9 is applied on or adjacent such a strip 10, which has an appearance different from the rest of the backsheet material 3, the identification of the location of the wetness indicator 9 is facilitated. Moreover the colour, tint or other appearance of the strip 10, may be an indicator of the product type, size, absorption capacity or the like. On or adjacent the strip 10 there can be further arranged symbols 11, codes or the like, which indicate the product type, size, absorption capacity or the like. By this it is easier for the nurse to find the correct type of product in the case where the articles have been taken out of their packages and placed on a shelf or the like in a storage space in a nursing home or the like.

Besides the strip 10 may also serve as an indicator of the longitudinal centre of the article so that it is easier to put on the article correctly. It may also act as a longitudinal centre indicator during the manufacturing process.

The strip 10 can extend in the longitudinal direction of the article, preferably centrally along the product, as is shown in Fig. 1. It is also possible that the strip 10 extends in the transverse direction of the article, as is shown in Fig. 3 and 4. In this case the strip 10 should be arranged at the part of the product intended to form a folding line 11 in th folded package condition of the article (see Fig. 4). In the case where the article is folded along two folding line there should be strips 10 at each folding line 11, so that independently of how the article is put on the shelf on strip 10 should be visible outwards.

The width of the strip 10 should be between 1 and 8 cm, preferably between 2 and 7 cm and more preferably between 3 and 6 cm.

The invention is of course not limited to the described and illustrated embodiments but a plurality of modifications are possible within the scope of the appended claims.

## Claims

1. Absorbent article such as a diaper and incontinence guard having a longitudinal and a transverse direction, comprising a liquid pervious topsheet (2), a liquid impervious backsheet (3) and an absorbent body (4) enclosed therebetween, at which on the inside of the liquid impervious backsheet (3), i e on the side adjacent the absorbent body, there is arranged a wetness indicator (9) in a certain pattern, which is visible through the backsheet material,
**characterized in,**
**that** the wetness indicator (9) is applied on or adjacent at least one strip (10) having a width and having a colour or tint different from the rest of the backsheet material (3).

2. Absorbent article as claimed in claim 1,
**characterized in,**
**that** the strip (10) is separate from the backsheet material (3) and is fastened on the inside thereof.

3. Absorbent article as claimed in claim 1,
characteri zed in,
that the strip (10) is a part of the backside material (3) which is coloured in another colour or tint than the rest of the backsheet material.

4. Absorbent article as claimed in any of the preceding claims,
**characterized in,**
**that** the colour or tint of the strip (10) is an indication of the product type, size, absorption capacity or the like of the article.

5. Absorbent article as claimed in any of the preceding claims,
**characterized in,**
**that** the strip (10) extends in the longitudinal direction of the article over the entire or at least an essential part of the length of the article.

6. Absorbent article as claimed in any of claims 1-4,
**characterized in,**
**that** the strip (10) extends in the transverse direction of the article and is applied at a part of the article that is intended to form a folding line in the folded packaging position of the article.

7. Absorbent article as claimed in any of the preceding claims,
**characterized in,**
**that** the width of the strip (10) is between 1 and 8 cm.

8. Absorbent article as claimed in any of the preceding claims,
**characterized in,**
**that** on or adjacent the strip (10) there is further printed symbols (11), codes or the like which indicate the product type, size, absorption capacity or the like of the article.

## Patentansprüche

1. Absorbierender Gegenstand, wie beispielsweise eine Windel und ein Inkontinenzschutz, mit einer Längs- und einer Querrichtung, umfassend eine flüssigkeitsdurchlässige Oberlage (2), eine flüssigkeitsundurchlässige Decklage (3) und einen Absorptionskörper (4), der dazwischen eingeschlossen ist, bei dem auf der Innenseite der flüssigkeitsundurchlässigen Deckschicht (3), d.h. auf der Seite benachbart dem Absorptionskörper, in einem gewissen Muster ein Feuchtigkeitsindikator (9) angeordnet ist, der durch das Deckmaterial sichtbar ist,
**dadurch gekennzeichnet,**
**dass** der Befeuchtungsindikator (9) auf oder benachbart wenigstens eines Streifens (10) mit einer Breite aufgebracht ist, der eine Farbe oder Färbung aufweist, die sich von dem Rest des Decklagenmaterials (3) unterscheidet.

2. Absorbierender Gegenstand nach Anspruch 1,
**dadurch gekennzeichnet,**
**dass** der Streifen (10) von dem Decklagenmaterial (3) getrennt und an der Innenseite davon befestigt ist.

3. Absorbierender Gegenstand nach Anspruch 1,
**dadurch gekennzeichnet,**
**dass** der Streifen (10) ein Teil des Decklagenmaterials (3) ist, das in einer anderen Farbe oder Färbung gestaltet ist als der Rest des Decklagenmaterials.

4. Absorbierender Gegenstand nach einem der vorstehenden Ansprüche,
**dadurch gekennzeichnet,**
**dass** die Farbe oder Färbung des Streifens (10) eine Indikation des Produkttyps, der Produktgröße, der Absorptionsfähigkeit oder ähnlichem des Gegenstands bereitstellt.

5. Absorbierender Gegenstand nach einem der vorstehenden Ansprüche,
**dadurch gekennzeichnet,**
**dass** sich der Streifen (10) in der Längsrichtung des Gegenstands über die gesamte oder wenigstens einen wesentlichen Teil der Länge des Gegenstands erstreckt.

6. Absorbierender Gegenstand nach Anspruch 1 bis 4,
**dadurch gekennzeichnet,**
**dass** sich der Streifen (10) in der Querrichtung des Gegenstands erstreckt und in einem Teil des Gegenstands aufgebracht ist, der dazu gedacht ist, in der gefalteten Packposition des Gegenstands eine Faltlinie zu bilden.

7. Absorbierender Gegenstand nach einem der vorstehenden Ansprüche,
**dadurch gekennzeichnet,**
**dass** die Breite des Streifens (10) zwischen 1 und 8 cm liegt.

8. Absorbierender Gegenstand nach einem der vorstehenden Ansprüche,
**dadurch gekennzeichnet,**
**dass** auf oder benachbart dem Streifen (10) weitere gedruckte Symbole (11), Kodierungen oder ähnliches vorgesehen sind, die den Produkttyp, die Produktgröße, die Absorptionsfähigkeit oder ähnliches des Gegenstands kennzeichnen.

## Revendications

1. Article absorbant tel qu'une couche-culotte et une protection contre l'incontinence, ayant un sens longitudinal et un sens transversal, comprenant une feuille supérieure (2) perméable aux liquides, une feuille arrière (3) imperméable aux liquides et un corps absorbant (4) enfermé entre celles-ci, sur lequel, sur l'intérieur de la feuille arrière (3) imperméable aux liquides, c'est-à-dire sur le côté adjacent au corps absorbant, est disposé un indicateur d'humidité (9) selon un certain motif, qui est visible à travers le matériau de la feuille arrière ;
**caractérisé en ce que**
l'indicateur d'humidité (9) est appliqué sur ou à proximité d'au moins une bande (10) ayant une certaine largeur et présentant une couleur ou teinte différente de celles du reste du matériau (3) de feuille arrière.

2. Article absorbant selon la revendication 1,
**caractérisé en ce que**
la bande (10) est séparée du matériau (3) de la feuille arrière et est fixée sur l'intérieur de celui-ci.

3. Article absorbant selon la revendication 1,
**caractérisé en ce que**
la bande (10) est une partie du matériau (3) du côté arrière qui est coloré dans une autre couleur ou teinte que celle du reste du matériau de la feuille arrière.

4. Article absorbant selon l'une quelconque des revendications qui précèdent,
**caractérisé en ce que**
la couleur ou teinte de la bande (10) est une indication du type de produit, de la taille, de la capacité d'absorption ou d'une caractéristique similaire à l'article.

5. Article absorbant selon l'une quelconque des revendications qui précèdent,
**caractérisé en ce que**
la bande (10) s'étend dans le sens longitudinal de l'article sur toute la longueur de l'article, ou au moins sur une partie essentielle de celle-ci.

6. Article absorbant selon l'une quelconque des revendications qui précèdent,
**caractérisé en ce que**
la bande (10) s'étend dans le sens transversal de l'article et **en ce qu'**elle est appliquée sur une partie de l'article qui est destinée à former une ligne de pliage dans l'article à l'état d'emballage plié.

7. Article absorbant selon l'une quelconque des revendications qui précèdent,
**caractérisé en ce que**
la largeur de la bande (10) est comprise entre 1 et 8 cm.

8. Article absorbant selon l'une quelconque des revendications qui précèdent,
**caractérisé en ce que**
sur la bande (10) ou sur une bande adjacente se trouvent en outre des symboles imprimés (11), des codes ou des inscriptions similaires qui indiquent le type de produit, la taille, la capacité d'absorption ou des caractéristiques similaires de l'article.
